# EUROPÄISCHE PATENTSCHRIFT

(11) **EP 2 536 277 B1**
(45) Veröffentlichungstag und Bekanntmachung des Hinweises auf die Patenterteilung: **20.01.2016**
(21) Anmeldenummer: 11703007.2
(22) Anmeldetag: 15.02.2011
(51) Int. Cl.: A01N 39/04, A01N 41/10, C07C 235/20, C07C 259/06, C07C 317/24, A01P 13/00, C07C 323/65

(54) **3-AMINOCARBONYL SUBSTITUIERTE BENZOYLCYCLOHEXANDIONE UND IHRE VERWENDUNG ALS HERBIZIDE**
3-AMINOCARBONYL SUBSTITUTED BENZOYLCYCLOHEXANDIONS AND THEIR APPLICATION AS HERBICIDES
BENZOYLCYCLOHEXANDIONE SUBSTITUÉE PAR 3-AMINOCARBONYLE ET SON UTILISATION EN TANT QU'HERBICIDE

(30) Priorität: 19.02.2010 EP 10001688
(43) Veröffentlichungstag der Anmeldung: 26.12.2012
(73) Patentinhaber: Bayer Intellectual Property GmbH, 40789 Monheim (DE)
(72) Erfinder: UENO, Chieko, 60323 Frankfurt (DE); DÖRNER-RIEPING, Simon, 61267 Neu-Anspach (DE); VAN ALMSICK, Andreas, 61184 Karben (DE); ROSINGER, Christopher, Hugh, 65719 Hofheim (DE); DITTGEN, Jan, 60316 Frankfurt (DE); FEUCHT, Dieter, 65760 Eschborn (DE); HÄUSER-HAHN, Isolde, 51375 Leverkusen (DE)
(86) Internationale Anmeldenummer: PCT/EP2011/052168
(87) Internationale Veröffentlichungsnummer: WO 2011/101321

(56) Entgegenhaltungen:
- EP-A2- 0 502 492
- WO-A1-02/081434
- WO-A1-03/022810
- WO-A2-2004/105482
- US-A- 4 816 066

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bereits bekannt, daß bestimmte Benzoylcyclohexandione herbizide Eigenschaften besitzen. So werden in WO 03/022810 Benzoylcyclohexandione beschrieben, deren Phenylring in 3-Position Aminocarbonyl substituiert ist. WO 2004/105482 A2 beschreibt Benorylcyclohexandione, deren Phenylring in 2-Position eine Trifluormethylgruppe und in 3-Position eine Alkoxyalkoxy-Gruppe trägt. EP 0 502 492 A2 beschreibt Benzoylcyclohexandione, deren Phenylring in 2-Position eine Trifluormethylgruppe und in 3-Position eine Halogenalkoxy-Gruppe trägt. US 4 816 066 A und WO 02/081434 A1 beschreiben Benzoylcyclohexandione, deren Phenylring jeweils in 2-Position eine Trifluormethylgruppe und in 3-Position verschiedene Reste trägt. Die aus dieser Schrift bekannten Verbindungen zeigen jedoch häufig eine nicht ausreichende herbizide Wirksamkeit oder eine nicht ausreichende Verträglichkeit gegenüber Kulturpflanzen.

Aufgabe der vorliegenden Erfindung ist daher die Bereitstellung von weiteren herbizid wirksamen Verbindungen mit - gegenüber den aus dem Stand der Technik bekannten Verbindungen - verbesserten Eigenschaften. Es wurde nun gefunden, daß 2-Benzoylcyclohexandione, deren Phenylring in 3-Position - über ein Sauerstoffatom gebunden - bestimmte Reste aus der Gruppe Aminocarbonylalkyl trägt, als Herbizide besonders gut geeignet sind.

Ein Gegenstand der vorliegenden Erfindung sind 3-Aminocarbonyl substituierte Benzoylcyclohexandione der Formel (I) oder deren Salze 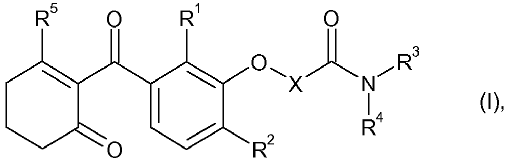 worin
- X: bedeutet Methylen;
- R¹: bedeutet Trifluormethyl;
- R²: bedeutet (C₁-C₆)-Alkyl-S(O)ₙ;
- R³ und R⁴: bedeuten unabhängig voneinander Wasserstoff, Methyl, Ethyl, Methoxy oder Ethoxy;
- R⁵: bedeutet Hydroxy;
- n: bedeutet 0,1 oder 2.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Sinngemäß gilt dies auch für Alkoxyreste.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen R⁵ für Hydroxy steht, können beispielsweise nach der in Schema 1 angegebenen Methode durch basenkatalysierte Umsetzung einer Verbindung der Formel (II), in der L^{a} für eine Fluchtgruppe wie Fluor oder Chlor steht, mit Alkoholen der Formel (III) hergestellt werden. Derartige Methoden werden unter anderem in Science of Synthesis (2007), 31, S. 609 ff. beschrieben.

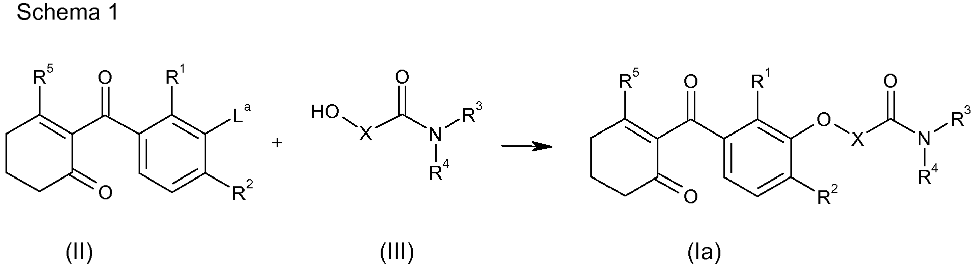

Verbindungen der Formel (II), können beispielsweise nach der in Schema 2 angegebenen Methode durch basenkatalysierte Umsetzung einer Verbindung der Formel (V), in der T für Halogen oder Hydroxy steht, mit Cyclohexandion (IV) in Anwesenheit einer Cyanid-Quelle hergestellt werden. Solche Methoden sind beispielsweise in EP0186117 beschrieben.

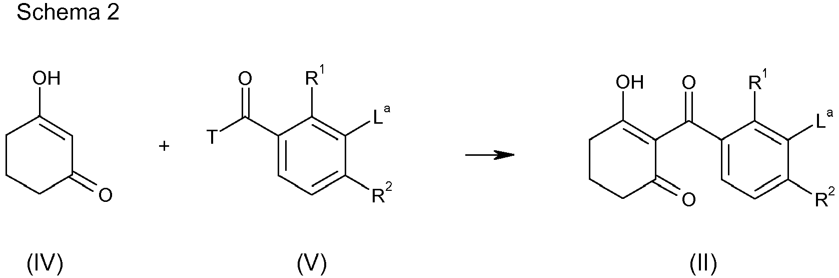

Verbindungen der Formel (III) können beispielsweise gemäß der in Schema 3 angegebenen Methode hergestellt werden. Die dort beschriebene Umsetzung von aktivierten Carbonsäure-Verbindungen der Formel (VI), worin L^{b} für eine Fluchtgruppe wie Chlor und G¹ für eine Hydroxy-Schutzgruppe wie zum Beispiel Benzyl steht, mit Aminen der Formel (VII) führt zunächst zu Amiden der Formel (VIII), die nach anschließender Abspaltung der Hydroxy-Schutzgruppe G¹ zu Alkoholen der Formel (III) umgesetzt werden können. Derartige Synthesen sind beispielsweise aus Organic Letters (2003), Vol. 5, No. 14, Seite 2539-2541 bekannt.

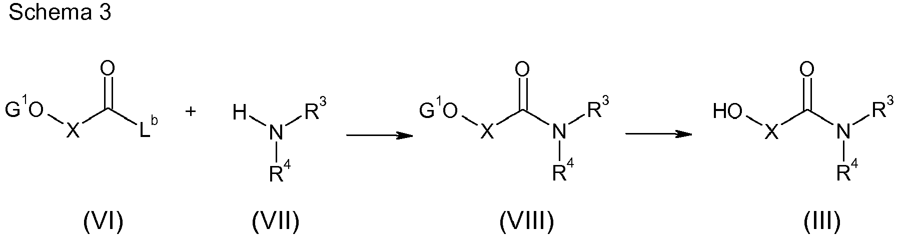

Gemäß den in Schema 4 angegebenen Methoden können Verbindungen der Formel (V) ausgehend von Verbindungen der Formel (Va), die beispielsweise in WO2008002853A2 beschrieben sind, hergestellt werden. Für den Fall, dass R² für (C₁-C₆)-Alkyl-S(O)ₙ steht, lässt sich ein Anilin der Formel (Va) zunächst diazotieren und das entstehende Diazoniumsalz anschließend mit entsprechenden Schwefelverbindungen zu Verbindungen der Formel (Vb) umsetzen. Derartige Methoden sind unter anderem in Science of Synthesis (2007), 31, Seite 984 folgende oder in Synthetic Communications (2001), 31, 12, Seite 1857-1861 beschrieben. Steht R² für Chlor lässt sich ein Anilin der Formel (Va) zunächst diazotieren und das entstehende Diazoniumsalz anschließend in einer Sandmeyer-Reaktion in Anwesenheit von Kupferchlorid zu Verbindungen der Formel (Vb) umsetzen, wie beispielsweise in Science of Synthesis (2007), 31, Seite 86 folgende beschrieben. Durch Brom-Lithium-Austausch an Verbindungen der Formel (Vb) durch Behandlung mit Lithiumverbindungen wie zum Beispiel n-Butyllithium und nachfolgender Umsetzung mit Elektrophilen wie Kohlendioxid gelangt man zu erfindungsgemäßen Verbindungen der Formel (V), in der in diesem Fall T für Hydroxy steht. Synthesen dieser Art werden zum Beispiel in Science of Synthesis (2003), 17 Seite 42 beschrieben. Durch Reaktion mit einem Oxidationsmittel, wie Wasserstoffperoxid, *m*-Chlorperoxybenzoesäure, Peroxyessigsäure und Kaliumperoxymonosulfat, werden weitere erfindungsgemäße Verbindungen der Formel (V) erhalten, in der R² für (C₁-C₆)-Alkyl-SO oder (C₁-C₆)-Alkyl-S(O)₂ steht. Solche Reaktionen sind beispielsweise beschrieben in J. Org. Chem. (1988), 53, 532 oder Tetrahedron Lett. (1981), 21, Seite 1287.

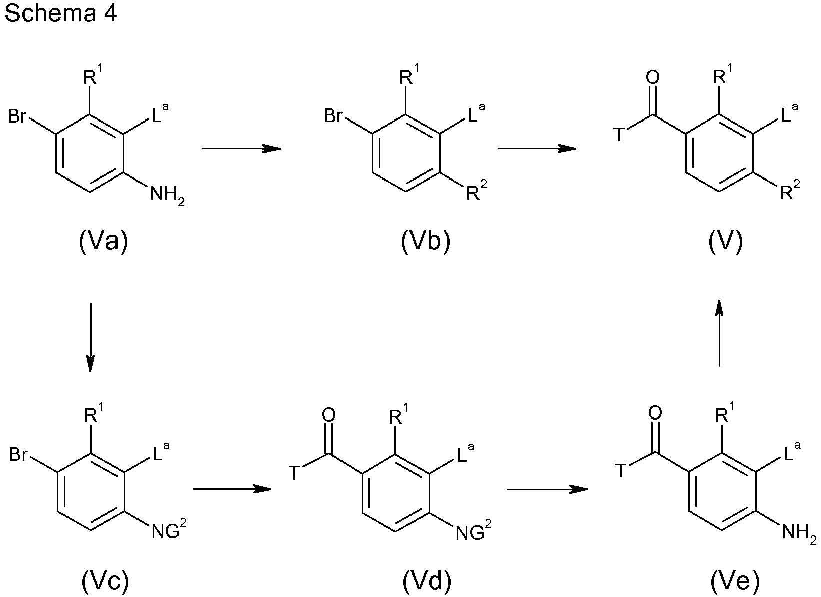

Verbindungen der Formel (V), in der T für Halogen steht können nach allgemein bekannten Methoden aus den analogen Hydroxy-Verbindungen durch Behandlung mit einem Halogenierungsmittel wie zum Beispiel Oxalylchlorid hergestellt werden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT^{™} (Glyphosate ALS Tolerant),
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259),
- transgene Kulturpflanzen mit modifizierter Fettsäurezusammensetzung (WO 91/13972),
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398),
- Transgene Kulturpflanzen, die pharmazeutisch oder diagnostisch wichtige Proteine produzieren ("molecular pharming"),
- transgene Kulturpflanzen, die sich durch höhere Erträge oder bessere Qualität auszeichnen,
- transgene Kulturpflanzen die sich durch eine Kombinationen z. B. der o. g. neuen Eigenschaften auszeichnen ("gene stacking").

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind. Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum^{™} GAT^{™} (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber

Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Besonders geeignete Safener sind Mefenpyr-diethyl, Isoxadifen-ethyl und Cyprosulfamid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, Bencarbazone, Benfluralin, Benfuresate, Bensulide, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyr-natrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifopbutyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indaziflam, Indolessigsäure (IAA), 4-Indol-3-ylbuttersäure (IBA), lodosulfuron, lodosulfuron-methyl-natrium, loxynil, Ipfencarbazone,Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazosulfuron, Methazole, Methiozolin, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, ZJ-0862 sowie die folgenden Verbindungen

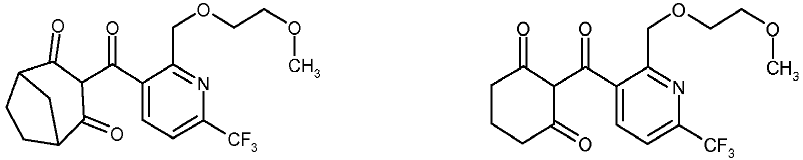

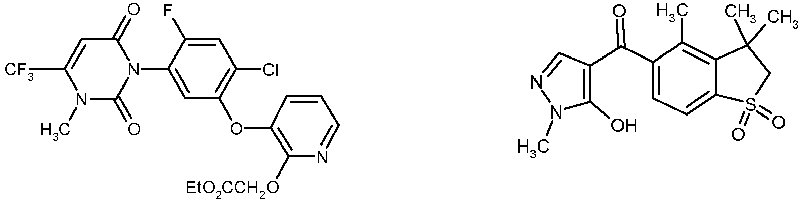

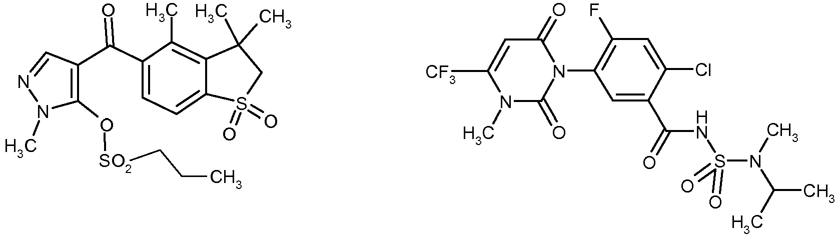

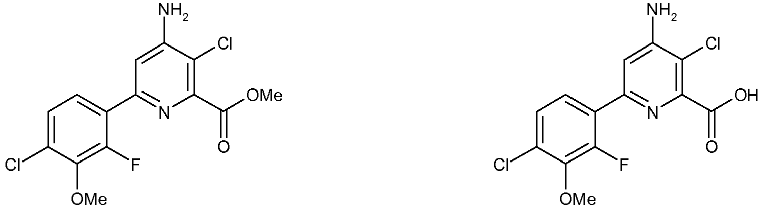

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Herstellung von 2-{3-[(2,6-Dioxocyclohexyl)carbonyl]-6-(methylsulfonyl)-2-(trifluormethyl)phenoxy}-N,N-diethylacetamid (Tabellenbeispiel Nr. 1-1829)

### Schritt 1: Synthese von 2-(Benzyloxy)-N,N-diethylacetamid

1.54 ml (9.7 mmol) Benzyloxyacetylchlorid und 0.92 ml (8.9 mmol) Diethylamin wurden bei 0°C in 35 ml CH₂Cl₂ gelöst. Das Reaktionsgemisch wurde 2 Stunden bei Raumtemperatur (RT) gerührt und anschließend mit verdünnter HCl-Lösung und gesättigter NaCl-Lösung gewaschen. Die organische Phase wurde über Na₂SO₄ getrocknet und vollständig eingeengt. 2-(Benzyloxy)-*N,N-*diethylacetamid wurde als farbloser Rückstand erhalten.
Ausbeute: 2.26 g (99% der Theorie)
¹H-NMR (CDCl₃): 7.28-7.40 (m, 5H), 4.61 (s, 2H), 4.16 (s, 2H), 3.39 (q, 2H), 3.29 (q, 2H), 1.12 (t, 6H).

### Schritt 2: Synthese von N,N-Diethyl-2-hydroxyacetamid

2.20 g (9.9 mmol) 2-(Benzyloxy)-*N,N-*diethylacetamid wurden in 45 ml Ethylacetat gelöst und mit 200 mg Pd/C (10%-ig) versetzt. Das Reaktionsgemisch wurde 24 Stunden in Wasserstoffatmosphäre gerührt und anschließend über Kieselgur filtriert. Das Filtrat wurde vollständig eingeengt und *N,N*-Diethyl-2-hydroxyacetamid als farbloser Rückstand erhalten.
Ausbeute: 1.22 g (82% der Theorie)
¹H-NMR (CDCl₃): 4.13 (s, 2H), 3.44 (q, 2H), 3.15 (q, 2H), 1.18 (t, 3H), 1.17 (t, 3H).

### Schritt 3: Synthese von 1-Brom-3-fluor-4-(methylsulfanyl)-2-(trifluormethyl)benzol

In 8.00 ml konzentrierte Schwefelsäure wurden unter Kühlung vorsichtig 1.12 g (16.3 mmol) Natriumnitrit eingetragen. Zu der klaren Lösung wurden bei 10-15°C 4.00 g (15.5 mmol) 4-Brom-2-fluor-3-(trifluormethyl)anilin gelöst in 30 ml Eisessig langsam zugetropft und 1.5 Stunden bei dieser Temperatur gerührt. 20 mg (0.31 mmol) Kupferpulver und 1.82 ml (20.2 mmol) Dimethyldisulfid wurden in 12 ml Eisessig vorgelegt und auf 45°C erwärmt. Zu dieser Lösung wurde die zuvor hergestellte Lösung des Diazoniumsalzes langsam zugetropft. Nach vollständiger Zugabe wurde das Reaktionsgemisch 4 Stunden bei 70°C gerührt. Anschließend wurde die Lösung in etwa 500 ml Wasser gegeben und mit CH₂Cl₂ extrahiert. Die organische Phase wurde mit Wasser, verdünnter NaHCO₃-Lösung, verdünnter HCl-Lösung und gesättigter NaCl-Lösung gewaschen. Die organische Phase wurde über Na₂SO₄ getrocknet und vollständig eingeengt. Das erhaltene Rohprodukt wurde säulenchromatographisch (Kieselgel, Gradient Heptan/Ethylacetat) getrennt. 1-Brom-3-fluor-4-(methylsulfanyl)-2-(trifluormethyl)benzol wurde als brauner Feststoff erhalten.
Ausbeute: 4.16 g (93% der Theorie)
¹H-NMR (CDCl₃): 7.46 (d, 1H), 7.21 (t, 1H), 2.48 (s, 3H).

### Schritt 4: Synthese von 3-Fluor-4-(methylsulfanyl)-2-(trifluormethyl)benzoesäure

21.0 g (72.98 mmol) 1-Brom-3-fluor-4-(methylsulfanyl)-2-(trifluormethyl)benzol wurden in 275 ml absolutem Diethylether gelöst und auf -78°C abgekühlt. 54.8 ml (87.59 mmol) einer 1.6M Lösung von *n*-Butyllithium in Hexan wurden langsam zugetropft und die entstehende Lösung für 60 Minuten bei -78°C gerührt. Anschließend wurde das Reaktionsgemisch auf frisch zerkleinertes Trockeneis gegeben und langsam auf RT erwärmt. Das Reaktionsgemisch wurde mit Wasser versetzt, mit 2M NaOH-Lösung basisch gestellt und mit Diethylether gewaschen. Die wässrige Phase wurde anschließend mit 5M HCl-Lösung angesäuert, wobei das Produkt ausfiel. Der Niederschlag wurde abgesaugt und im Vakuum getrocknet.
Ausbeute: 16.32 g (84% der Theorie)
¹H-NMR (CDCl₃): 7.35-7.42 (m, 2H), 2.50 (s, 3H).

### Schritt 5: Synthese von 3-Fluor-4-(methylsulfonyl)-2-(trifluormethyl)benzoesäure

30.00 g (118.0 mmol) 3-Fluor-4-(methylsulfanyl)-2-(trifluormethyl)benzoesäure wurden in 350 ml Essigsäure gelöst und mit 1.04 g (472.1 mmol) Natriumwolframat versetzt. Das Reaktionsgemisch wurde auf 60°C erwärmt und langsam 35%-ige Wasserstoffperoxid-Lösung zugetropft, so dass die Temperatur nicht über 80°C steigt. Nach der Zugabe wurde noch 60 Minuten bei 60°C gerührt. Anschließend wurde das Reaktionsgemisch mit 500 ml Wasser verdünnt. Die wässrige Phase wurde mit Natriumchlorid gesättigt und mit Ethylacetat extrahiert. Die vereinigten organischen Phasen wurden mit Natriumbisulfit-Lösung gewaschen über Na₂SO₄ getrocknet und vollständig eingeengt. 3-Fluor-4-(methylsulfonyl)-2-(trifluormethyl)benzoesäure wurde als beiger Feststoff erhalten.
Ausbeute: 33.00 g (98% der Theorie)
¹H-NMR (CDCl₃): 8.26 (t, 1H), 7.63 (d, 1H), 3.30 (s, 3H).

### Schritt 6: Synthese von 2-[3-Fluor-4-(methylsulfonyl)-2-(trifluormethyl)benzoyl]cyclo-hexan-1,3-dion

3.00 g (10.5 mmol) 3-Fluor-4-(methylsulfonyl)-2-(trifluormethyl)benzoesäure wurden in 30 ml CH₂Cl₂ gelöst und mit 5 Tropfen Dimethylformamid versetzt. 1.14 ml, 13.1 mmol) Oxalylchlorid wurden zugetropft und die Lösung für 15 Minuten unter Rückfluss erhitzt. Anschließend wurde das Reaktionsgemisch eingeengt und der Rückstand kurz im Vakuum getrocknet. Der Rückstand wurde in 30 ml Dichlormethan aufgenommen und 1.29 g (11.5 mmol) 1,3-Cyclohexandion sowie 2.92 ml (21.0 mmol) Triethylamin zugefügt. Das Reaktionsgemisch wurde 60 Minuten bei RT gerührt. Anschließend wurde mit 1M HCl-Lösung gewaschen, die organische Phase über Na₂SO₄ getrocknet und vollständig eingeengt.

Der so erhaltene Enol-Ester wurde in 30 ml Acetonitril gelöst und mit 2.19 ml (15.7 mmol) Triethylamin, 210 µl (1.6 mmol) Trimethylsilylcyanid und 205 mg (3.1 mmol) Kaliumcyanid versetzt. Das Reaktionsgemisch wurde über Nacht bei RT gerührt und anschließend das Lösemittel abgezogen. Der Rückstand wurde mit 1N H₂SO₄-Lösung versetzt und mit Diethylether extrahiert. Die organische Phase wurde über Na₂SO₄ getrocknet und vollständig eingeengt. Man erhielt 2-[3-Fluor-4-(methylsulfonyl)-2-(trifluormethyl)benzoyl]cyclohexan-1,3-dion als gelben Feststoff.
Ausbeute: 3,82 g (86% der Theorie)
¹H-NMR (CDCl₃): 16.2 (bs, 1H), 8.15 (dd, 1H), 7.08 (d, 1H), 3.38 (s, 3H), 2.82 (t, 2H), 2.42 (t, 2H), 2.07 (quin, 2H).

### Schritt 7: Synthese von 2-{3-[(2,6-Dioxocyclohexyl)carbonyl]-6-(methylsulfonyl)-2-(trifluormethyl)phenoxy}-N,N-diethylacetamid 150 mg (0.39 mmol) 2-[3-Fluor-4-(methylsulfonyl)-2-(trifluormethyl)benzoyl]cyclohexan-1,3-dion und 56.9 mg (0.43 mmol) N,N-Diethyl-2-hydroxyacetamid wurden in 5 ml absolutem

Dimethylformamid gelöst. 56.8 mg (1.42 mmol) Natriumhydrid (Suspension 60% in Mineralöl) wurden zugegeben und das entstandene Reaktionsgemisch über Nacht bei RT gerührt. Nach vorsichtiger Zugabe von 10 ml Wasser wurde mit Diethylether gewaschen. Die wässrige Phase wurde anschließend mit 2M HCl-Lösung sauer gestellt und mit Dichlormethan extrahiert. Die organische Phase wurde über Na₂SO₄ getrocknet und vollständig eingeengt. 2-{3-[(2,6-Dioxocyclohexyl)carbonyl]-6-(methylsulfonyl)-2-(trifluormethyl)phenoxy}-*N,N*-diethylacetamid wurde als gelblicher Feststoff erhalten.
Ausbeute: 210 mg (99% der Theorie)
¹H-NMR (CDCl₃): 8.01 (d, 1H), 7.07 (d, 1H), 4.90-5.00 (m, 2H), 3.49 (s, 3H), 3.44 (q, 2H), 3.12 (q, 2H), 2.82 (t, 2H), 2.42 (t, 2H), 2.08 (quin, 2H), 1.19 (t, 3H), 1.18 (t, 3H).

Die in nachfolgender Tabelle 1 aufgeführten Beispiele wurden analog oben genannten Methoden hergestellt beziehungsweise sind analog oben genannten Methoden erhältlich. Diese Verbindungen sind ganz besonders bevorzugt.

Die verwendeten Abkürzungen bedeuten:
Et = Ethyl Me = Methyl

**Tabelle 1:**

| Nr. | X | R¹ | R² | -NR³R⁴ | R⁵ | Physikalische Daten |
|---|---|---|---|---|---|---|
| 1-1825 | -CH₂- | CF₃ | SO₂Me | -NH₂ | OH | |
| 1-1826 | -CH₂- | CF₃ | SO₂Me | -NHMe | OH | |
| 1-1827 | -CH₂- | CF₃ | SO₂Me | -NMe₂ | OH | CDCl₃: 16.32 (bs, 1H), 8.21 (d, 1H), 7.07 (d, 1H), 4.90-5.00 (m, 2H), 3.50 (s, 3H), 3.01 (s, 3H), 2.90 (s, 3H), 2.82 (t, 2H), 2.42 (t, 2H), 2.07 (quin, 2H) |
| 1-1828 | -CH₂- | CF₃ | SO₂Me | -NHEt | OH | |
| 1-1829 | -CH₂- | CF₃ | SO₂Me | -NEt₂ | OH | CDCl₃: 8.21 (d, 1H), 7.07 (d, 1H), 4.90-4.98 (m, 2H), 3.50 (s, 3H), 3.43 (q, 2H), 3.12 (q, 2H), 2.81 (t, 2H), 2.41 (t, 2H), 2.06 (quin, 2H), 1.17 (t, 3H), 1.16 (t, 3H) |
| 1-1832 | -CH₂- | CF₃ | SO₂Me | -NMeEt | OH | CDCl₃: 16.31 (bs, 1H), 8.20 (d, 1H), 7.07 (d, 1H), 4.86-5.00 (m, 2H), 3.49 (s, 3H), 3.15 (q, 1H), 2.98 (s, 1H), 2.78-2.85 (m, 5H) 2.42 (t, 2H), 2.08 (quin, 2H), 1.12-1.20 (m, 3H) |
| 1-1838 | -CH₂- | CF₃ | SO₂Me | -N(Me)OMe | OH | CDCl₃: 16.32 (bs, 1H), 8.22 (d, 1H), 7.08 (d, 1H), 5.00-5.10 (m, 2H), 3.67 (s, 3H), 3.48 (s, 3H); 3.25 (s, 3H), 2.81 (t, 2H), 2.42 (t, 2H), 2.08 (quin, 2H) |
| 1-1839 | -CH₂- | CF₃ | SO₂Me | -N(Me)OEt | OH | |
| 1-1841 | -CH₂- | CF₃ | SO₂Me | -N(Et)OMe | OH | |
| 1-1842 | -CH₂- | CF₃ | SO₂Me | -N(Et)OEt | OH | |
| 1-2281 | -CH₂- | CF₃ | SO₂Et | -NH₂ | OH | |
| 1-2282 | -CH₂- | CF₃ | SO₂Et | -NHMe | OH | |
| 1-2283 | -CH₂- | CF₃ | SO₂Et | -NMe₂ | OH | CDCl₃: 16.33 (bs, 1H), 8.19 (d, 1H), 7.08 (d, 1H), 4.87-5.00 (m, 2H), 3.72 (q, 2H), 3.00 (s, 3H); 2.84 (s, 3H), 2.80 (t, 2H), 2.41 (t, 2H), 2.07 (quin, 2H), 1.22 (t, 3H) |
| 1-2284 | -CH₂- | CF₃ | SO₂Et | -NHEt | OH | |
| 1-2285 | -CH₂- | CF₃ | SO₂Et | -NEt₂ | OH | CDCl₃: 16.35 (bs, 1H), 8.20 (d, 1H), 7.07 (d, 1H), 4.88-4.98 (m, 2H), 3.73 (q, 2H), 3.45 (q, 2H), 3.13 (q, 2H), 2.80 (t, 2H), 2.41 (t, 2H), 2.06 (quin, 2H), 1.22 (t, 3H),+ 1.17 (t, 3H), 1.16 (t, 3H) |
| 1-2288 | -CH₂- | CF₃ | SO₂Et | -NMeEt | OH | CDCl₃: 16.33 (bs, 1H), 8.19 (d, 1H), 7.07 (d, 1H), 4.83-5.00 (m, 2H), 3.72 (q, 2H), 3.48 (q, 1H), 3.15 (q, 1H), 2.98 (s, 1H), 2.77-2.85 (m, 4H) 2.40 (t, 2H), 2.07 (quin, 2H), 1.11-1.20 (m, 3H), 1.20-1.25 (m, 3H) |
| 1-2294 | -CH₂- | CF₃ | SO₂Et | -N(Me)OMe | OH | CDCl₃: 16.32 (bs, 1H), 8.19 (d, 1H), 7.07 (d, 1H), 5.01-5.08 (m, 2H), 3.69 (q, 2H), 3.65 (s, 3H); 3.22 (s, 3H), 2.80 (t, 2H), 2.41 (t, 2H), 2.07 (quin, 2H), 1.22 (t, 3H) |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und 7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Vergleichsbeispiele

Für die biologischen Vergleichsbeispiele wurden folgende erfindungsgemäßen und aus dem nächstliegenden Stand der Technik (WO 03/022810) bekannten Verbindungen verwendet:

| erfindungsgemäß | aus WO 03/022810 bekannt |
|---|---|
| 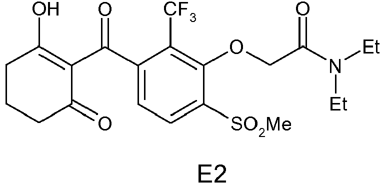 | 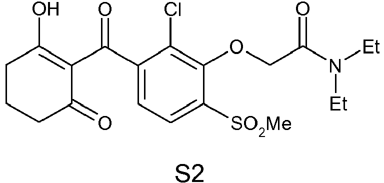 |
| 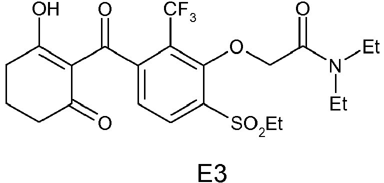 | 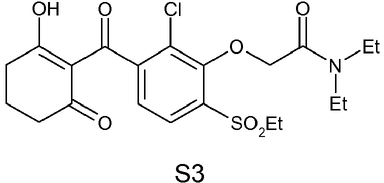 |
| 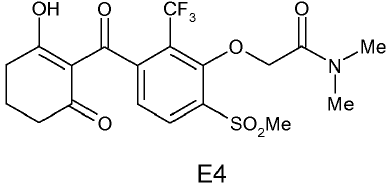 | 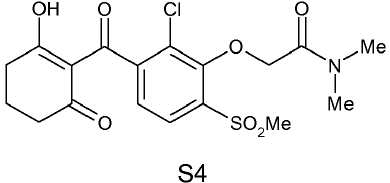 |
| 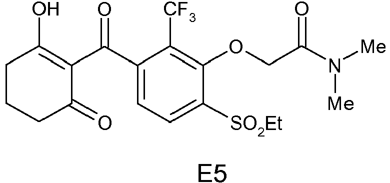 | 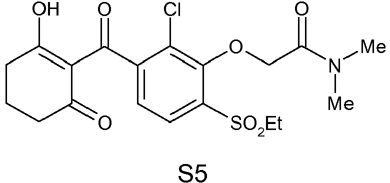 |
| 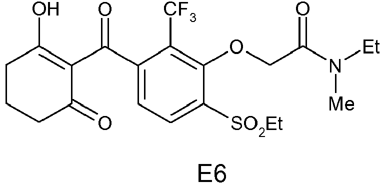 | 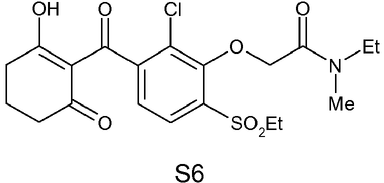 |
| 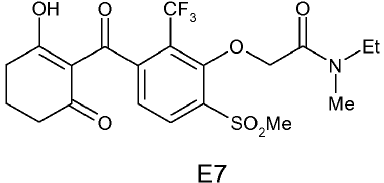 | 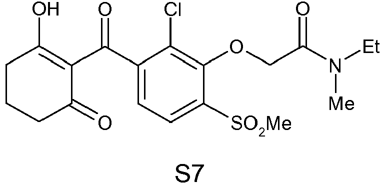 |
| 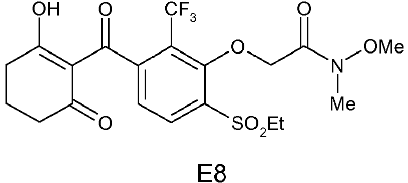 | 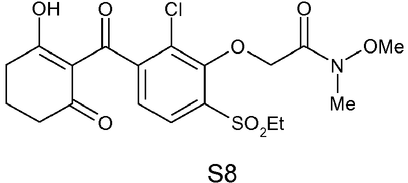 |
| 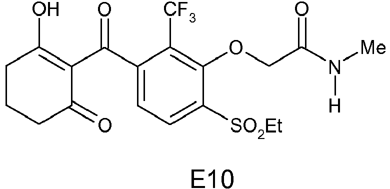 | 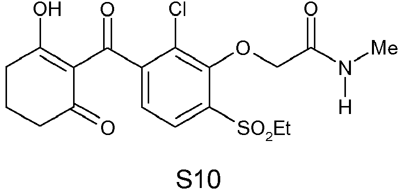 |

Die in folgenden Tabellen verwendeten Abkürzungen bedeuten:

| | | | |
|---|---|---|---|
| ABUTH | Abutilon theophrasti | ALOMY | Alopecurus myosuroides |
| AMARE | Amaranthus retroflexus | AVEFA | Avena fatua |
| ECHCG | Echinochloa crus galli | MATIN | Matricaria inodora |
| PHBPU | Pharbitis purpureum | POLCO | Fallopia convolvulus |
| STEME | Stellaria media | VERPE | Veronica persica |

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen und aus dem Stand der Technik bekannten Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen, -- = nicht getestet). Die Ergebnisse der Vergleichstabelle A zeigen, dass die erfindungsgemäßen Verbindungen höhere herbizide Wirkung haben als die aus dem nächstliegenden Stand der Technik bekannten Verbindungen größtmöglicher struktureller Ähnlichkeit.

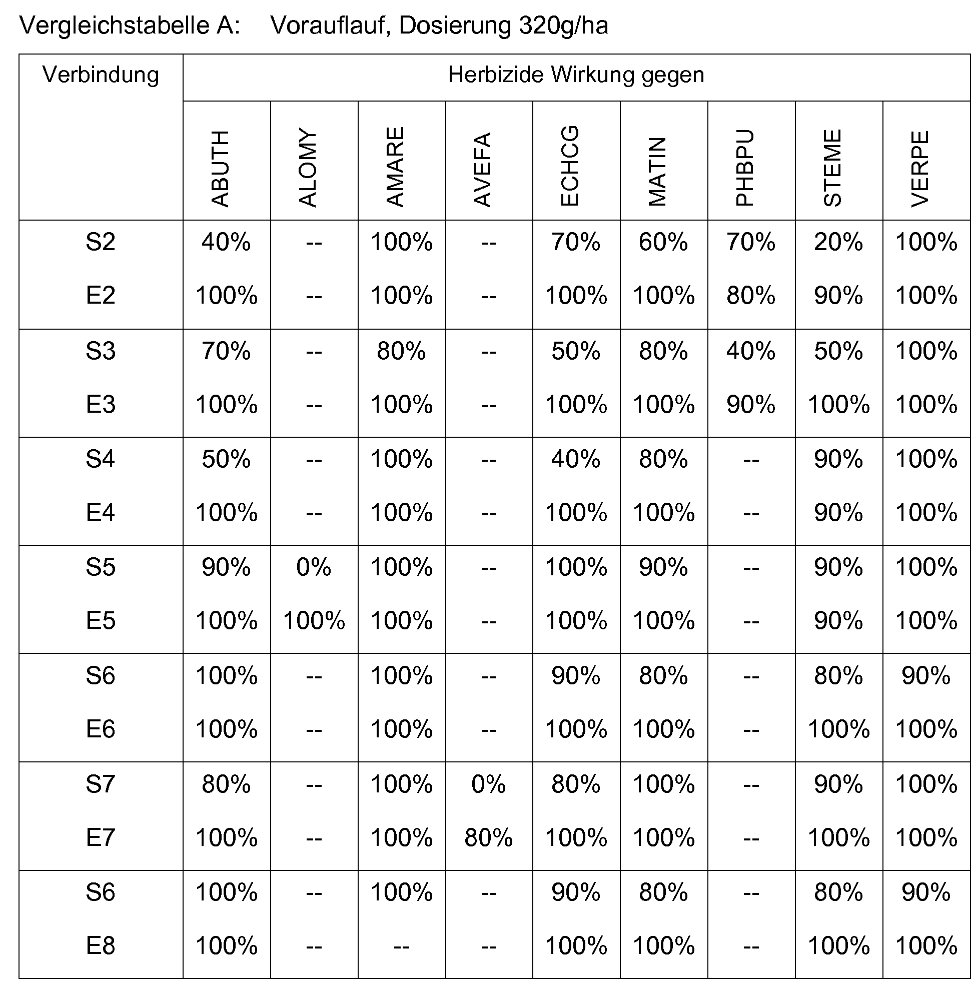

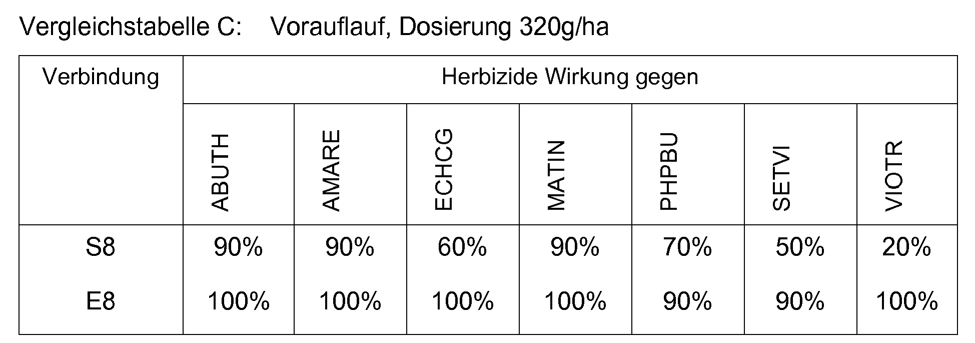

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = Kontrollpflanzen, -- = nicht getestet). Die Ergebnisse der Vergleichstabelle B zeigen, dass die erfindungsgemäßen Verbindungen höhere herbizide Wirkung haben als die aus dem nächstliegenden Stand der Technik bekannten Verbindungen größtmöglicher struktureller Ähnlichkeit.

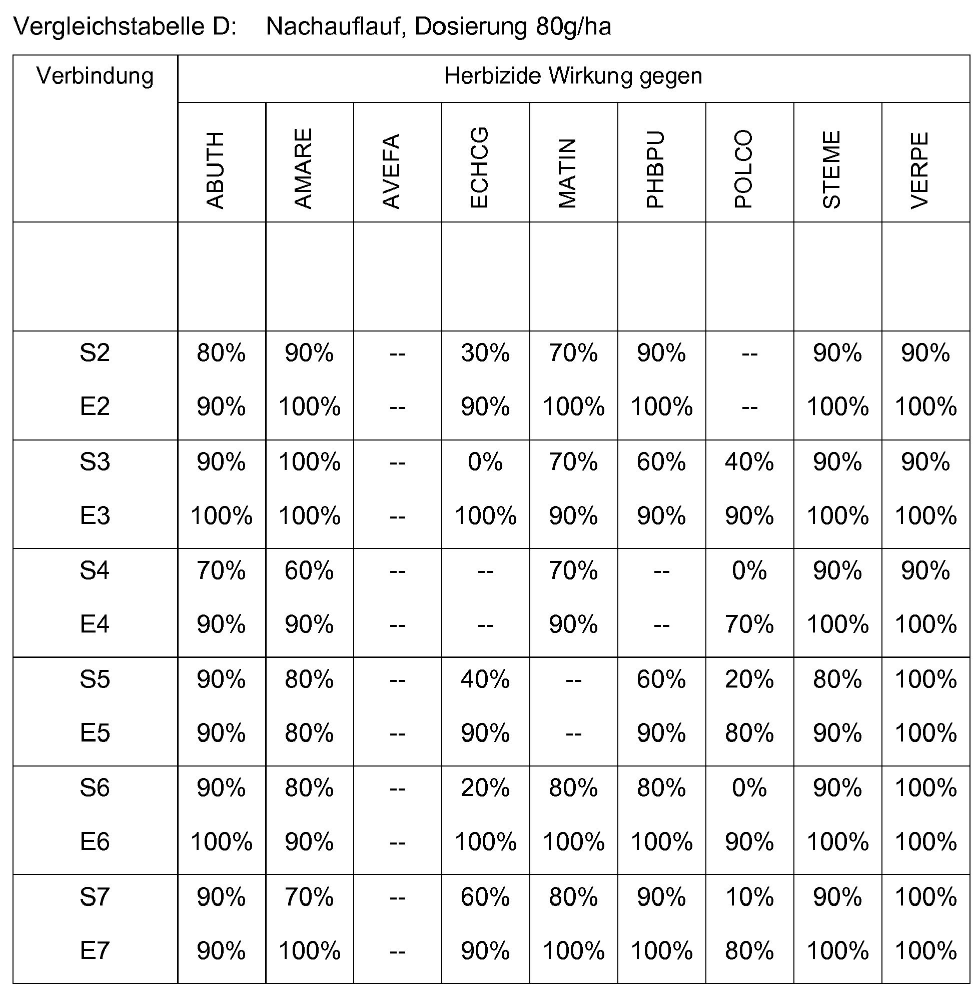

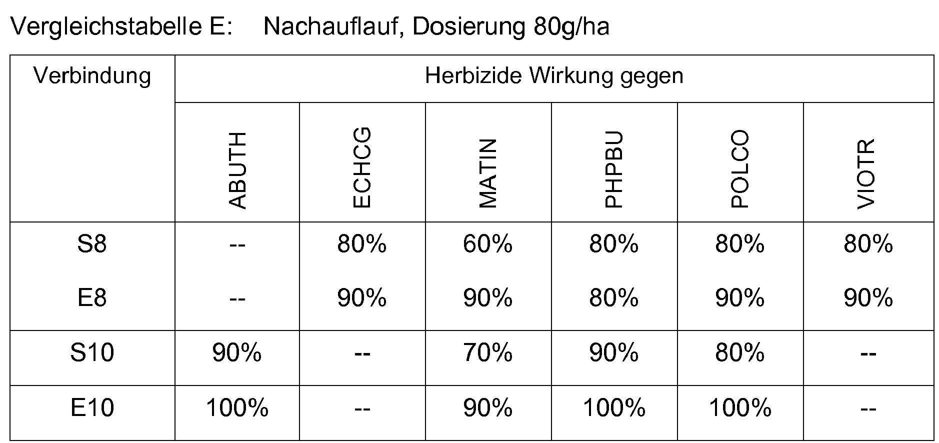

## Patentansprüche

1. 3-Aminocarbonyl substituierte Benzoylcyclohexandione der Formel (I) oder deren Salze 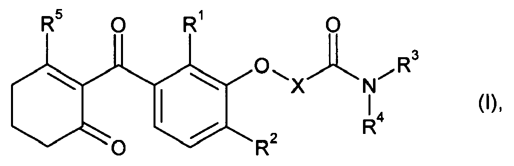 worin
X bedeutet Methylen;
R¹ bedeutet Trifluormethyl;
R² bedeutet (C₁-C₆)-Alkyl-S(O)ₙ;
R³ und R⁴ bedeuten unabhängig voneinander Wasserstoff, Methyl, Ethyl, Methoxy oder Ethoxy;
R⁵ bedeutet Hydroxy;
n bedeutet 0, 1 oder 2.

2. Herbizide Mittel, **gekennzeichnet durch** einen herbizid wirksamen Gehalt an mindestens einem 3-Aminocarbonyl substituierten Benzoylcyclohexandion der Formel (I) gemäß Anspruch 1.

3. Herbizide Mittel nach Anspruch 2 in Mischung mit Formulierungshilfsmitteln.

4. Herbizide Mittel nach Anspruch 2 oder 3 enthaltend mindestens einen weiteren pestizid wirksamen Stoff aus der Gruppe Insektizide, Akarizide, Herbizide, Fungizide, Safenern und Wachstumsregulatoren.

5. Herbizide Mittel nach Anspruch 4 enthaltend mindestens einen Safener.

6. Herbizide Mittel nach Anspruch 5 enthaltend Mefenpyr-diethyl, Isoxadifen-ethyl oder Cyprosulfamid.

7. Verfahren zur Bekämpfung unerwünschter Pflanzen, **dadurch gekennzeichnet, dass** man eine wirksame Menge mindestens einer Verbindung der Formel (I) gemäß Anspruch 1 oder eines herbiziden Mittels gemäß einem der Ansprüche 2 bis 6 auf die Pflanzen oder auf den Ort des unerwünschten Pflanzenwachstums appliziert.

8. Verwendung von Verbindungen der Formel (I) gemäß Anspruch 1 oder eines herbiziden Mittels gemäß einem der Ansprüche 2 bis 6 zur Bekämpfung unerwünschter Pflanzen.

9. Verwendung nach Anspruch 8, **dadurch gekennzeichnet, dass** die Verbindungen der Formel (I) zur Bekämpfung unerwünschter Pflanzen in Kulturen von Nutzpflanzen eingesetzt werden.

10. Verwendung nach Anspruch 9, **dadurch gekennzeichnet, dass** die Nutzpflanzen transgene Nutzpflanzen sind.

## Claims

1. A 3-aminocarbonyl-substituted benzoylcyclohexanedione of the formula (I) or a salt thereof 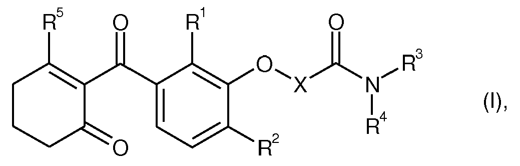 in which
X is methylene;
R¹ is trifluoromethyl;
R² is (C₁-C₆)-alkyl-S(O)ₙ;
R³ and R⁴ independently of one another are hydrogen, methyl, ethyl, methoxy or ethoxy;
R⁵ is hydroxyl;
n is 0, 1 or 2.

2. A herbicidal composition which comprises a herbicidally effective amount of at least one 3-aminocarbonyl-substituted benzoylcyclohexanedione of the formula (I) as claimed in claim 1.

3. The herbicidal composition as claimed in claim 2 in a mixture with formulation auxiliaries

4. The herbicidal composition as claimed in claim 2 or 3 which comprises at least one further pesticidally active compound from the group of the insecticides, acaricides, herbicides, fungicides, safeners and growth regulators.

5. The herbicidal composition as claimed in claim 4 which comprises at least one safener.

6. The herbicidal composition as claimed in claim 5 which comprises mefenpyr-diethyl, isoxadifen-ethyl or cyprosulfamide.

7. A method for controlling unwanted plants which comprises applying an effective amount of at least one compound of the formula (I) as claimed in claim 1 or of a herbicidal composition as claimed in any of claims 2 to 6 to the plants or the site of the unwanted vegetation.

8. The use of compounds of the formula (I) as claimed in claim 1 or of a herbicidal composition as claimed in any of claims 2 to 6 for controlling unwanted plants.

9. The use as claimed in claim 8, wherein the compounds of the formula (I) are used for controlling unwanted plants in crops of useful plants.

10. The use as claimed in claim 9, wherein the useful plants are transgenic useful plants.

## Revendications

1. Benzoylcyclohexanediones substituées par 3-aminocarbonyle de formule (I) ou leurs sels 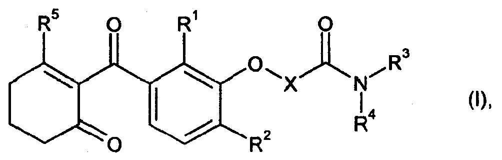 dans laquelle
X signifie méthylène ;
R¹ signifie trifluorométhyle ;
R² signifie (C₁-C₆)-alkyl-S(O)ₙ ;
R³ et R⁴ signifient, indépendamment l'un de l'autre, hydrogène, méthyle, éthyle, méthoxy ou éthoxy ;
R⁵ signifie hydroxy ;
n vaut 0, 1 ou 2.

2. Agents herbicides, **caractérisés par** une teneur active en tant qu'herbicide en au moins une benzoylcyclohexanedione substituée par 3-aminocarbonyle de formule (I) selon la revendication 1.

3. Agents herbicides selon la revendication 2 en mélange avec des adjuvants de formulation.

4. Agents herbicides selon la revendication 2 ou 3 contenant au moins une autre substance active en tant que pesticide du groupe formé par les insecticides, les acaricides, les herbicides, les fongicides, les phytoprotecteurs et les régulateurs de croissance.

5. Agents herbicides selon la revendication 4 contenant au moins un phytoprotecteur.

6. Agents herbicides selon la revendication 5, contenant du méfenpyr-diéthyl, de l'isoxadifen-éthyl ou du cyprosulfamide.

7. Procédé pour lutter contre des plantes non souhaitées, **caractérisé en ce qu'**on applique une quantité active d'au moins un composé de formule (I) selon la revendication 1 ou d'un agent herbicide selon l'une quelconque des revendications 2 à 6 sur les plantes ou sur le lieu de la croissance non souhaitée des plantes.

8. Utilisation de composés de formule (I) selon la revendication 1 ou d'un agent herbicide selon l'une quelconque des revendications 2 à 6 pour lutter contre des plantes non souhaitées.

9. Utilisation selon la revendication 8, **caractérisée en ce que** les composés de formule (I) sont utilisés pour lutter contre des plantes non souhaitées dans les cultures de plantes utiles.

10. Utilisation selon la revendication 9, **caractérisée en ce que** les plantes utiles sont des plantes utiles transgéniques.
